# FASCICULE DE BREVET EUROPEEN

(11) **EP 1 405 600 B1**
(45) Date de publication et mention de la délivrance du brevet: **15.07.2009**
(21) Numéro de dépôt: 03292285.8
(22) Date de dépôt: 16.09.2003
(51) Int. Cl.: A61B 10/00

(54) **Dispositif pour prélever un échantillon corporel**
Vorrichtung zur Entnahme einer Körperprobe
Apparatus for taking a bodily sample

(30) Priorité: 03.10.2002 FR 0212234
(43) Date de publication de la demande: 07.04.2004
(73) Titulaire: Coloplast A/S, 3050 Humlebæk (DK)
(72) Inventeur: Blondeau, Jérôme, 75013 Paris (FR)

(56) Documents cités:
- US-A- 4 699 154
- US-A- 4 958 625
- US-A- 5 842 999
- US-A- 5 951 489

## Description

La présente invention concerne un dispositif pour prélever un échantillon de tissu ou d'organe corporel sur un être humain ou animal à des fins d'examen.

Le prélèvement d'échantillon, désigné généralement par le terme de biopsie, est devenu un acte médical de plus en plus pratiqué puisqu'il permet entre autres opérations de diagnostiquer des états maladifs à la suite de différents examens de l'échantillon prélevé et d'atteindre, sans lésion, des organes profonds tels que le foie et les reins.

Aussi, de nombreux dispositifs de prélèvement ont-ils été développés pour répondre à la demande avec pour objectif de faciliter leur utilisation par les praticiens et de garantir une fiabilité de fonctionnement élevée.

On connaît déjà de nombreux dispositifs pour prélever des échantillons corporels et ils comportent généralement :
- une aiguille dont l'extrémité distale forme un évidement apte à recevoir ledit échantillon ;
- une canule entourant coaxialement ladite aiguille, lesdites aiguille et canule pouvant coulisser l'une par rapport à l'autre ;
- des coulisseaux associés respectivement auxdites aiguille et canule ;
- des ressorts associés respectivement auxdits coulisseaux ;
- un boîtier de préhension de forme allongée, définissant un logement interne à l'intérieur duquel sont agencés en série, selon un axe longitudinal dudit boîtier, lesdits coulisseaux pouvant coulisser entre une position avant dans le boîtier, pour laquelle lesdites aiguille et canule sont en position de repos, prêtes à être armées pour un tir de prélèvement, et une position arrière, pour laquelle lesdites aiguille et canule sont en position rétractée armée, prêtes pour ledit tir ;
- un bouton de commande pour amener en position arrière lesdits coulisseaux à l'encontre desdits ressorts respectifs ;
- des moyens de blocage desdits coulisseaux en position arrière ; et
- un mécanisme de déclenchement pour effacer lesdits moyens de blocage et entraîner, sous l'action desdits ressorts, le déplacement en position avant desdits coulisseaux et le tir de prélèvement desdites aiguille et canule.

De tels dispositifs sont par exemple enseignés par les brevets européens EP-0 238 461 et EP-0 435 986.

Ces dispositifs décrits nécessitent l'usage des deux mains du praticien pour charger simultanément les coulisseaux portant respectivement l'aiguille et la canule, à l'encontre des ressorts, ce qui n'est pas toujours facile à réaliser.

L'invention a pour objet de proposer un dispositif de prélèvement dont la conception, structurellement simple, permet notamment au praticien de faire passer lesdites aiguille et canule en position armée, d'une seule main.

A cet effet, le dispositif de prélèvement du type préalablement décrit est remarquable, selon l'invention, en ce que lesdits coulisseaux comportent des butées qui sont transversalement décalées l'une par rapport à l'autre et en ce que ledit bouton de commande comprend un ergot mobile transversalement sous l'action de moyens de déplacement, et agissant séquentiellement sur lesdites butées décalées pour amener l'un après l'autre lesdits coulisseaux en position arrière.

Ainsi, grâce à l'invention, un seul bouton de commande permet le chargement séquentiel de l'aiguille et de la canule qui s'effectue en deux mouvements distincts : un premier mouvement de translation du bouton depuis une position initiale, qui amène par exemple le coulisseau à canule en position armée et, après retour dudit bouton en position initiale, un second mouvement de translation qui amène le coulisseau à aiguille en position armée.

L'utilisation du dispositif est simple et aisée puisque le praticien peut armer celui-ci d'une seule main à l'aide de son pouce.

Par exemple, lesdits moyens de déplacement peuvent comprendre un ressort disposé transversalement entre ledit bouton et ledit ergot et permettant le passage de ce dernier d'une position rentrée pour laquelle l'un desdits coulisseaux est déplacé en position arrière via sa butée, à une position sortie pour laquelle l'autre coulisseau est déplacé en position arrière via sa butée décalée, et une rampe inclinée, prévue à l'intérieur dudit boîtier et qui ramène ledit ergot de sa position sortie vers sa position rentrée, lors du retour en position initiale dudit bouton.

Avantageusement, ladite rampe inclinée se termine par un bord d'extrémité latéral sur lequel s'applique, en position initiale dudit bouton, ledit ergot comprimant son ressort, et qui est situé au même niveau que la butée du coulisseau à déplacer en premier. Ainsi, l'ergot est maintenu en position rentrée et engage la butée du coulisseau correspondant aussitôt le déplacement du bouton de commande. L'ergot est lié audit bouton, par exemple, par une liaison à glissière et peut coulisser transversalement via celle-ci sous l'action des moyens de déplacement.

En particulier, ledit coulisseau à canule et son ressort sont situés du côté avant dudit boîtier et sont amenés en premier en position arrière, armée via ledit ergot, tandis que ledit coulisseau à aiguille et son ressort sont coaxialement situés du côté arrière dudit boîtier et sont déplacés en second en position arrière, armée, le déplacement desdits coulisseaux et ressorts étant limité par des pattes fixes solidaires dudit boîtier.

De préférence, ledit bouton de commande est monté longitudinalement coulissant à travers une ouverture oblongue dudit boîtier et un ressort disposé longitudinalement relie ledit boîtier audit bouton pour rappeler spontanément ce dernier dans sa position initiale, contre le bord avant correspondant de ladite ouverture.

Dans un mode préféré de réalisation, lesdits moyens de blocage comportent au moins une patte à crochet élastiquement déformable issue de chaque coulisseau, et une butée fixe correspondante prévue à l'intérieur dudit boîtier et sur laquelle s'engage la patte à crochet du coulisseau correspondant quand celui-ci arrive en position arrière.

En ce qui concerne ledit mécanisme de déclenchement dudit tir de prélèvement, il comporte avantageusement, sur ledit boîtier, une gâchette avant et une gâchette arrière actionnables indépendamment l'une de l'autre et agissant sur lesdits moyens de blocage. Le praticien peut se servir de l'une ou l'autre desdites gâchettes pour déclencher le tir. De préférence, lesdites gâchettes avant et arrière sont reliées mécaniquement entre elles par une tige de liaison agencée à l'intérieur dudit boîtier.

Dans un mode préféré de réalisation, ladite gâchette arrière comprend un bouton-poussoir avec ressort de rappel et muni d'une patte agencée en saillie dans ledit boîtier pour libérer lesdits moyens de blocage dudit coulisseau à aiguille, et ledit coulisseau à aiguille est équipé, de plus, d'une patte agencée en saillie pour agir sur lesdits moyens de blocage dudit coulisseau à canule, par suite de son déplacement vers la position avant. Ainsi, l'éjection du coulisseau à aiguille a tout d'abord lieu, puis celle du coulisseau à canule, ce qui permet de recueillir l'échantillon corporel entre les déplacements successifs des coulisseaux en position avant.

Quant à ladite gâchette avant, elle peut comprendre un levier pivotant autour d'un axe dudit boîtier orthogonal à son axe longitudinal, ladite tige de liaison reliant ledit levier de la gâchette avant audit bouton-poussoir de la gâchette arrière. Avantageusement, le dispositif comprend une sécurité pour rendre inopérationnel ledit mécanisme de déclenchement. Par exemple, elle consiste en une encoche ménagée dans ledit boîtier et dans laquelle peut être reçue ladite gâchette avant par suite d'un déplacement transversal. Le mécanisme de déclenchement est alors immobilisé.

Par ailleurs, ledit boîtier se compose, de préférence, de deux demi-coques assemblées entre elles le long d'un plan longitudinal.

Les figures du dessin annexé feront bien comprendre comment l'invention peut être réalisée. Sur ces figures, des références identiques désignent des éléments semblables.

Les figures 1, 2 et 3 sont respectivement des vues extérieures en plan, de dessus et latérale d'un exemple de réalisation du dispositif de prélèvement selon l'invention.

La figure 4 est une vue en perspective éclatée dudit dispositif montrant ses différents composants.

Les figures 5A, 5B, 5C, 5D, 5E, 5F, 5G et 5H sont des vues en coupe longitudinale dudit dispositif montrant les différentes étapes de fonctionnement, à savoir :
- coulisseaux à aiguille et à canule en position repos ;
- premier déplacement du bouton de commande pour l'armement du coulisseau à canule ;
- retour du bouton de commande en position initiale ;
- second déplacement du bouton de commande pour l'armement du coulisseau à aiguille ;
- retour du bouton de commande en position initiale ;
- commande du tir par le mécanisme de déclenchement et action sur les moyens de blocage du coulisseau à aiguille ;
- sortie du coulisseau à aiguille et action sur les moyens de blocage du coulisseau à canule ; et
- sortie du coulisseau à canule.

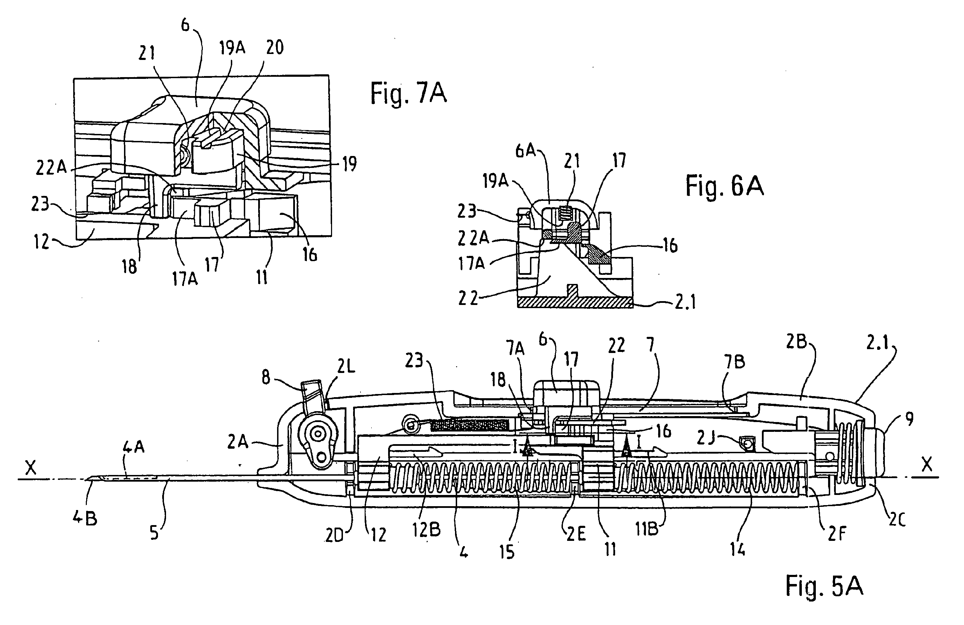

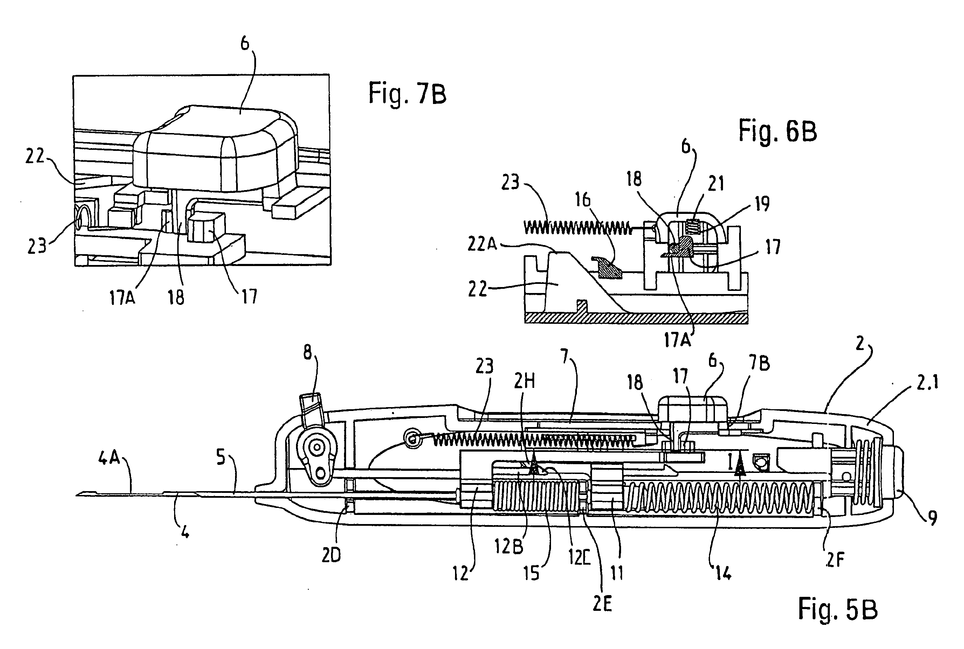

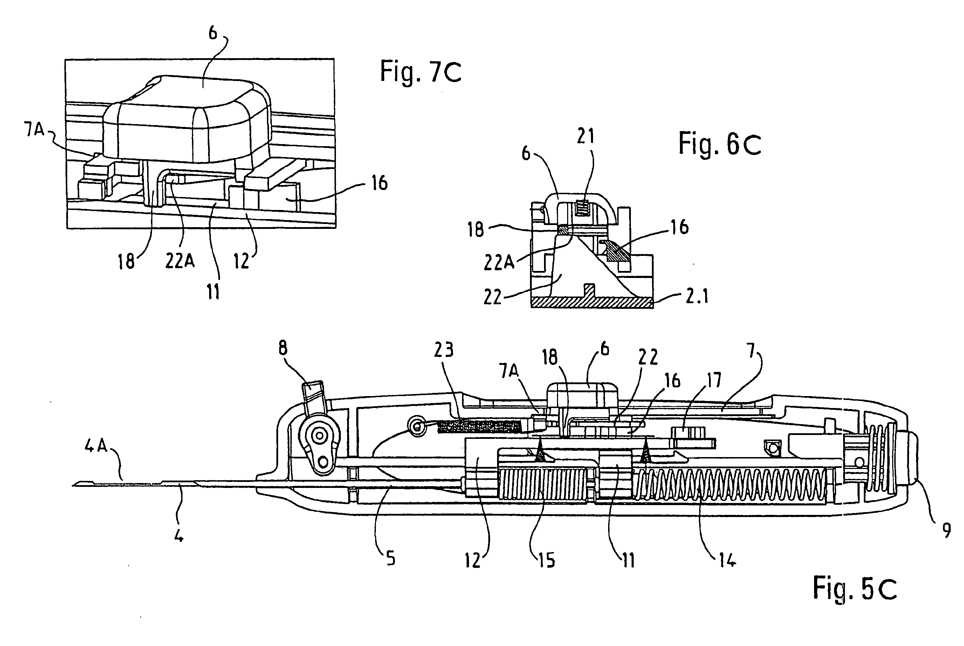

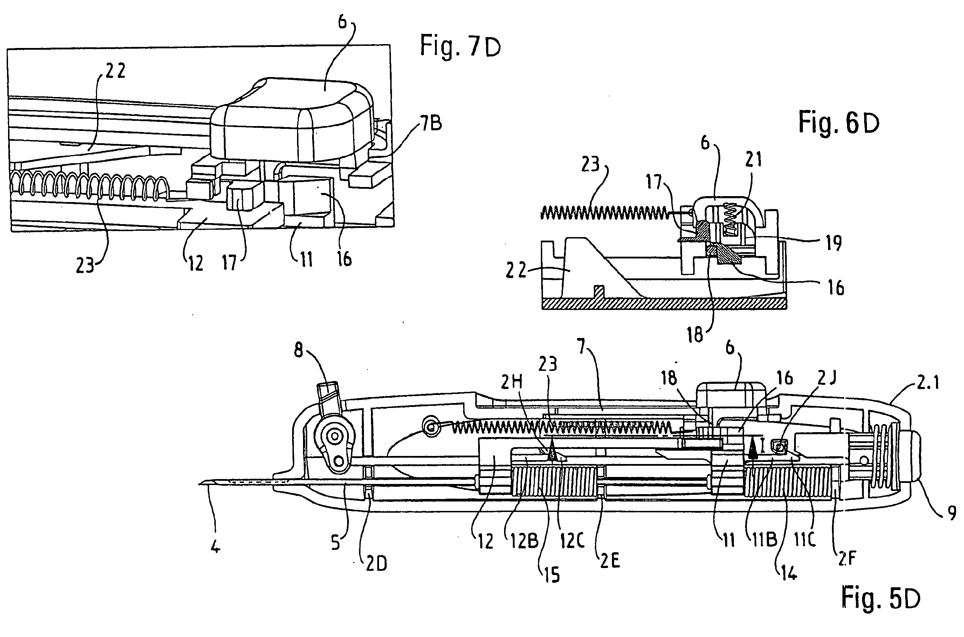

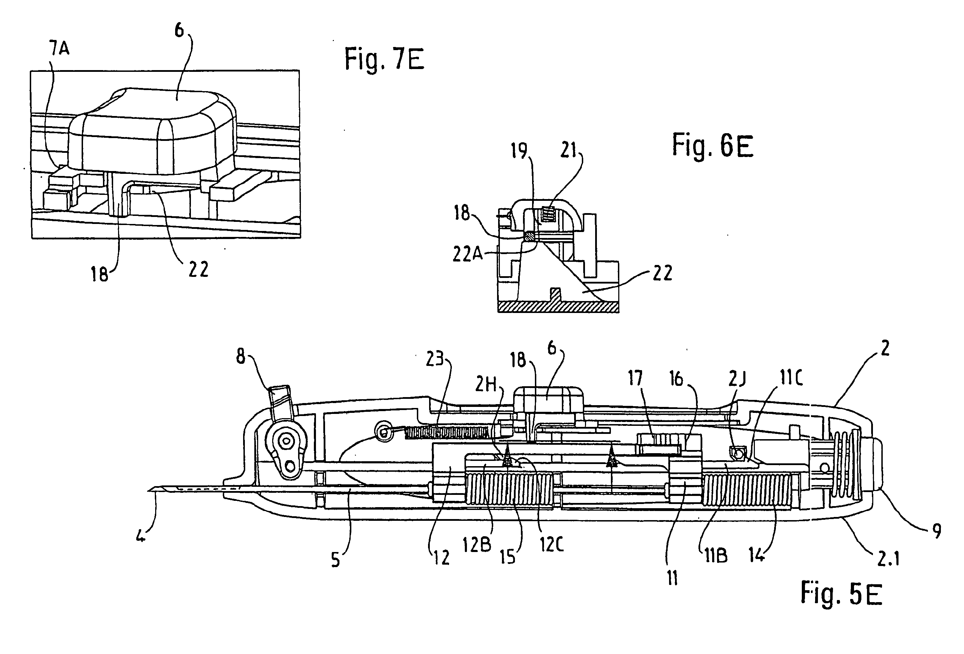

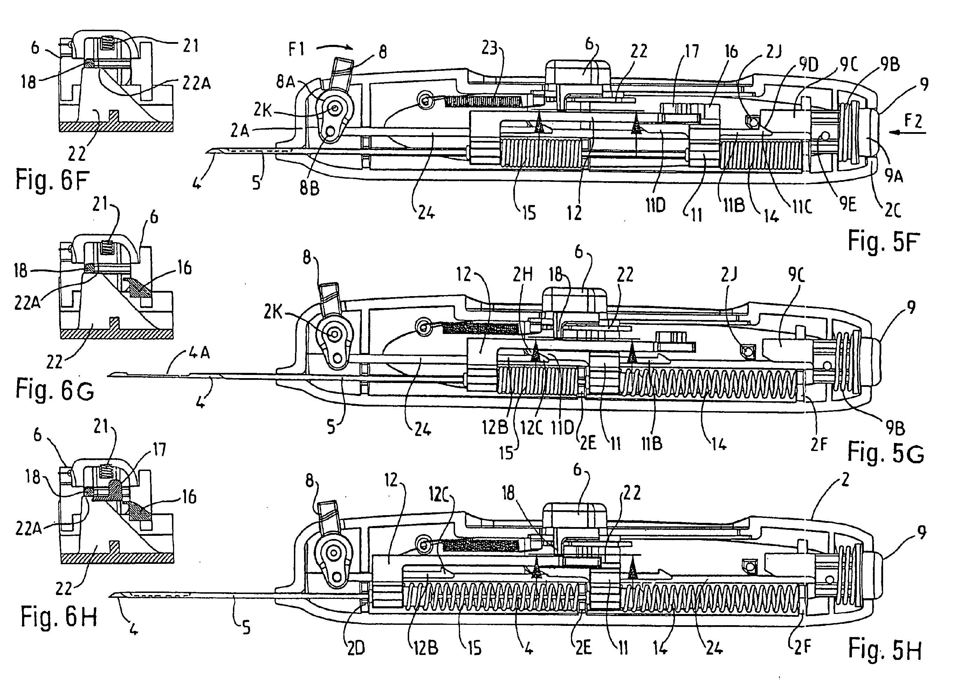

Les figures 6A, 6B, 6C, 6D, 6E, 6F, 6G et 6H sont des vues en coupe selon la ligne I-I des figures 5A, 5B, 5C, 5D, 5E, 5F, 5G et 5H montrant les différentes étapes de fonctionnement du bouton de commande dudit dispositif.

Les figures 7A, 7B, 7C, 7D et 7E sont des vues en perspective, délimitées par un cadre rectangulaire dudit bouton de commande agissant sur les butées des coulisseaux dans les positions occupées par le dispositif sur les figures 5A à 5E respectivement.

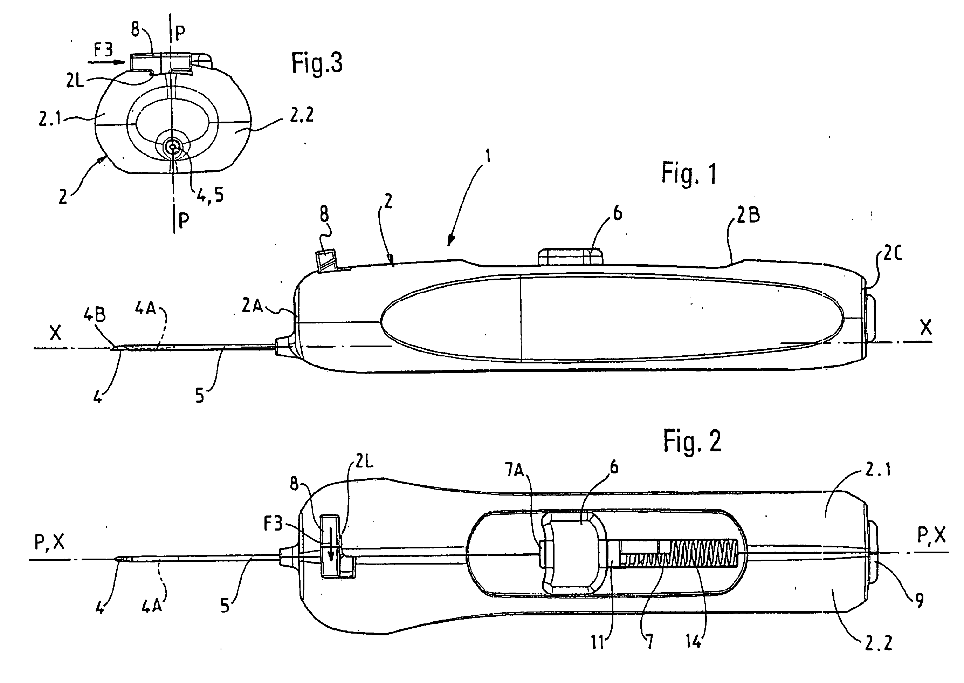

Le dispositif de prélèvement 1 d'un échantillon tissulaire corporel, montré sur les figures 1 à 3, comporte un boîtier de préhension 2 analogue à une poignée et dont la forme extérieure, sensiblement parallélépipédique arrondie, est anatomique, permettant au praticien de saisir convenablement et d'utiliser aisément le dispositif d'une main.

De la face avant 2A du boîtier émergent une aiguille 4 avec un évidement 4A et une canule 5 de prélèvement entourant coaxialement l'aiguille, à l'exception de la pointe d'introduction biseautée 4B, tandis qu'un bouton de commande 6 est accessible de la face de dessus 2B du boîtier, à travers une ouverture 7 ménagée dans celui-ci. Egalement, on voit sur ces figures que la gâchette avant 8 et la gâchette arrière 9 d'un mécanisme de déclenchement du tir desdites aiguille et canule sont accessibles de l'extérieur du boîtier et sont respectivement prévues sur le dessus du boîtier, près de sa face avant 2A, et sur sa face arrière 2C.

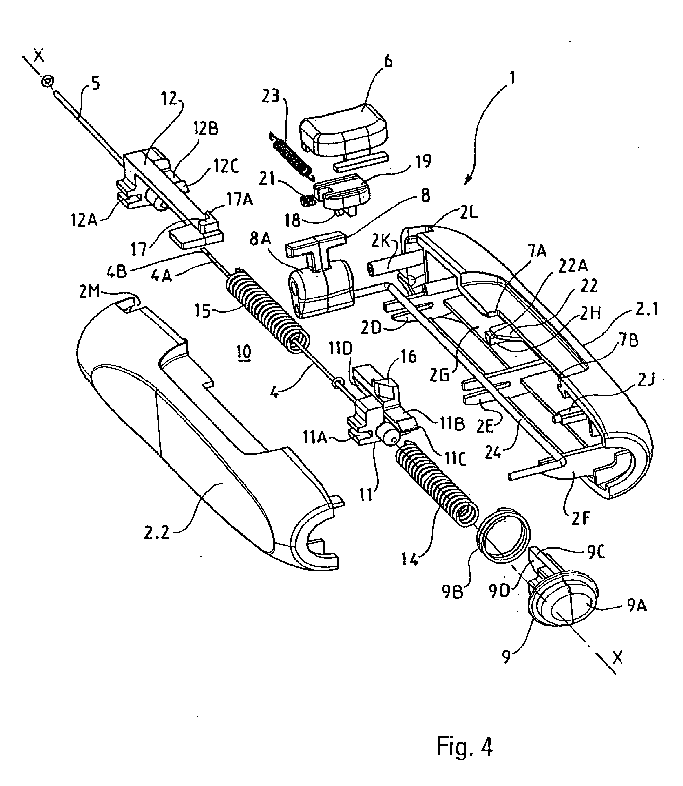

Comme le montre plus particulièrement la figure 4, le boîtier de préhension 2 est constitué de deux demi-coques 2.1, 2.2 assemblées entre elles pour définir un plan longitudinal médian P-P dudit boîtier, et un logement intérieur 10 à l'intérieur duquel sont montés les différents composants du dispositif de prélèvement 1 qui seront décrits ci-après en regard des figures 4, 5A, 6A et 7A.

Dans le logement interne 10 du boîtier 2, sont prévus deux coulisseaux 11, 12 agencés en série et portant respectivement l'aiguille 4 et la canule 5 selon un même axe longitudinal X-X. Dans le mode de réalisation illustré, le coulisseau 12 à canule 5 se trouve à l'avant du boîtier 2, tandis que le coulisseau 11 à aiguille 4 est situé derrière, c'est-à-dire tourné vers l'arrière du boîtier, son aiguille 4 traversant la canule 5 et le coulisseau associé 12 jusqu'à la sortie de sa pointe biseautée distale 4B de la canule. Des ressorts 14, 15 sont également associés respectivement aux coulisseaux en étant agencés suivant l'axe longitudinal X-X des coulisseaux. Ainsi, le coulisseau 12 à canule est en butée contre une patte avant fixe 2D du boîtier sous l'action du ressort 15 disposé entre le coulisseau et une patte intermédiaire fixe 2E du boîtier, et le coulisseau 11 à aiguille est pressé contre la patte intermédiaire fixe 2E, de l'autre côté, sous l'action du ressort 14 disposé entre le coulisseau et une troisième patte arrière fixe 2F du boîtier. Ces ressorts sont du type à compression et identiques. Ces trois pattes font par exemple saillie de la demi-coque 2.1 du boîtier, transversalement à son plan longitudinal P-P.

Ainsi, sous l'action de ces ressorts, lesdits coulisseaux 11, 12 occupent une position avant dans le boîtier 2, pour laquelle les aiguille 4 et canule 5 sont en position de repos initiale, non armée, largement sortie de la face avant 2A du boîtier.

Bien évidemment, les coulisseaux 11, 12 coopèrent, dans leur déplacement, avec des guides 2G issus des demi-coques 2.1 et 2.2 et s'engageant dans des rainures latérales respectives 11A, 12A de ceux-ci.

Par un déplacement vers l'arrière, parallèle à l'axe X-X, le bouton de commande 6 permet d'amener en position arrière les coulisseaux 11, 12, à l'encontre des ressorts respectifs 14, 15, position arrière pour laquelle les aiguille 4 et canule 5 sont alors en position rétractée armée, prêtes pour le tir de prélèvement, comme on le verra ultérieurement en regard de la figure 5D. Avantageusement, pour amener séquentiellement les coulisseaux en position arrière, ces derniers sont munis de butées respectives 16, 17 qui sont transversalement décalées l'une par rapport à l'autre, et le bouton de commande comprend un ergot 18 mobile transversalement, c'est-à-dire perpendiculairement au plan P-P, sous l'action de moyens de déplacement et qui peut agir séquentiellement sur les butées.

Comme le montrent les figures 4, 5A, 6A et 7A, la butée 17 est dressée verticalement à l'extrémité arrière du coulisseau 12 à canule et présente, par exemple, une forme coudée en L dans laquelle peut s'engager l'ergot mobile 18 associé au bouton de commande 6. De même, l'autre butée 16 est dressée verticalement sur le coulisseau 11 à aiguille, en étant située sensiblement à la même hauteur que la butée 17 du coulisseau 12 (figures 5A, 7A), mais décalée transversalement de ladite butée 17 (figures 6A, 7A). La butée 16 du coulisseau à aiguille est également coudée pour un engagement sûr et fiable dudit ergot 18. Ce dernier fait saillie verticalement vers le bas dudit bouton 6 pour venir juste au niveau des coulisseaux et il est issu d'une embase 19 logée à l'intérieur du bouton et montée transversalement coulissante dans celui-ci par une liaison à glissière 20.

Les moyens de déplacement de l'ergot 18 comprennent avantageusement un ressort 21 disposé transversalement contre une paroi latérale 6A du bouton et un évidement 19A ménagé dans l'embase, et une rampe inclinée 22 prévue à l'intérieur du boîtier et solidaire de la demi-coque 2.1 dont elle est issue.

Ainsi, le ressort 21 du type à compression assure le passage de l'ergot 18 d'une position rentrée, pour laquelle le coulisseau 12 est déplacé par sa butée 17 vers sa position arrière, à une position sortie, pour laquelle l'autre coulisseau 11 est déplacé vers l'arrière via sa butée 16 décalée transversalement par rapport à celle du coulisseau 12. Et la rampe 22 ramène ledit ergot 18 en position rentrée, comprimant le ressort 21, lors du retour en position initiale du bouton 6.

On voit notamment sur les figures 6A, 7A que la rampe inclinée 22 se termine par un bord d'extrémité 22A parallèle à l'axe X-X, sur lequel s'applique l'ergot en position initiale du bouton, et qui est avantageusement situé dans le prolongement du côté correspondant 17A de la butée 17 en L.

On remarque également que le bouton de commande 6 est maintenu en position initiale par un ressort de traction 23 le reliant à la demi-coque 2.1 du boîtier et l'amenant contre le bord avant 7A de l'ouverture oblongue 7 dudit boîtier.

Par ailleurs, le dispositif 1 comporte aussi des moyens de blocage pour immobiliser les coulisseaux 11, 12 lorsqu'ils occupent la position arrière. Pour cela, chaque coulisseau 11, 12 comprend, dans cet exemple de réalisation, une patte longitudinale élastiquement déformable 11B, 12B terminée par un crochet 11C, 12C, apte à s'encliqueter avec une butée fixe correspondante 2J, 2H faisant transversalement saillie de la demi-coque 2.1 du boîtier.

En ce qui concerne le mécanisme de déclenchement, la gâchette avant 8 et la gâchette arrière 9 peuvent être actionnées indépendamment l'une de l'autre selon la préférence du praticien, tout en étant avantageusement reliées mécaniquement entre elles pour agir sur les moyens de blocage 11B, 11C, 2J - 12B, 12C, 2H en position arrière des coulisseaux.

Structurellement, la gâchette avant 8 est issue d'un levier 8A monté pivotant autour d'un axe 2K situé en partie avant de la demi-coque et disposé orthogonalement à l'axe X-X ou au plan longitudinal P-P du boîtier 2. Quant à la gâchette arrière 9, elle est du type à bouton-poussoir 9A à déplacement longitudinal, avec un ressort de rappel 9B prévu entre celui-ci et la face arrière 2C du boîtier, et muni d'une patte 9C faisant saillie longitudinalement à l'intérieur dudit boîtier. Cette patte 9C est destinée à venir au contact des moyens de blocage 11B, 11C pour les effacer et libérer le coulisseau arrière 11, lequel comporte également une patte longitudinale 11D, opposée à celle 11B, pour agir sur les moyens de blocage 12B, 12C du coulisseau 12 dans le boîtier, comme on le verra ultérieurement.

Le levier pivotant 8A de la gâchette avant et le bouton-poussoir 9A de la gâchette arrière sont reliés par une tige ou tringle 24 courant à l'intérieur du boîtier et s'engageant par ses extrémités dans des trous correspondants 8B, 9E prévus dans le levier 8A et la patte 9C du bouton-poussoir. Ainsi, une action sur l'une quelconque des gâchettes entraîne le déplacement de la patte 9C agissant sur les moyens de blocage.

On remarque par ailleurs, sur les figures 2 et 3, que la gâchette avant 8 est légèrement décalée transversalement par rapport au plan longitudinal P-P du boîtier 1. Ce décalage transversal constitue une sécurité pour le mécanisme de déclenchement qui ne peut être rendu actif que si le praticien le ramène dans le plan longitudinal P-P. Pour cela, cette sécurité est définie par une encoche 2L prolongeant transversalement l'ouverture 2M ménagée dans le dessus du boîtier pour le passage du levier 8A de la gâchette avant, lequel levier peut coulisser transversalement sur son axe 2K pour être amené dans l'encoche 2L ou sorti de celle-ci par le praticien. Cette gâchette avant 8 (et, donc, le mécanisme de déclenchement) n'est rendue opérationnelle que si le praticien la déplace transversalement vers le plan longitudinal selon la flèche F3.

Le fonctionnement du dispositif de prélèvement 1 selon l'invention sera maintenant décrit.

Le dispositif se trouve initialement dans la configuration illustrée sur les figures 1, 2, 5A, 6A et 7A, pour laquelle :
- les coulisseaux 11, 12 occupent la position avant dans le boîtier, en butée contre les pattes avant et intermédiaire respectives 2B, 2E, sous l'action des ressorts 14, 15, position pour laquelle les canule 5 et aiguille 4 sont en position initiale sortie, de repos ; la pointe biseautée de la canule 5 recouvrant l'évidement 4A de l'aiguille ;
- le bouton de commande 6 est en position initiale avant par l'action du ressort 23, contre le bord avant 7A de l'ouverture du boîtier 2 ;
- l'ergot 18 se trouve en contact avec le bord d'extrémité latéral 22A de la rampe inclinée ; et
- le mécanisme de déclenchement 8, 9 est inactif.

Quand le praticien souhaite procéder à l'armement du dispositif 1, il recule, à l'aide de son pouce, le bouton de commande 6 jusqu'à ce qu'il vienne en butée contre le bord arrière 7B de l'ouverture 7. Comme le montrent les figures 5B, 6B et 7B, dès le commencement du déplacement longitudinal vers l'arrière du bouton, l'ergot 18 s'engage dans la butée coudée 17 du coulissement 12, laquelle est située dans le prolongement du bord d'extrémité 22A de la rampe 22. L'ergot 18 reste sur sa position transversale initiale avec son ressort comprimé puisqu'il s'applique contre le côté 17A de la butée. Ainsi, le coulisseau 12 est entraîné et passe de sa position avant à sa position arrière, en comprimant son ressort 15, jusqu'à l'engagement du crochet 12C de sa patte élastiquement déformable 12B avec la butée correspondante 2H du boîtier. La canule 5, qui a reculé avec son coulisseau, se trouve alors en position armée. On découvre sur la figure 5B l'évidement de prélèvement 4A de l'aiguille 4.

Quand le bouton de commande 6 revient de sa position arrière vers sa position avant via son ressort 23, comme le montrent les figures 5C, 6C et 7C, l'ergot 18 quitte la butée 17 du coulisseau 12, de sorte qu'il se déplace transversalement en position sortie par l'action du ressort 21 qui se détend et déplace l'embase 19 via la liaison 20. Puis, quand l'ergot touche la rampe inclinée 22, il suit celle-ci et reprend sa position rentrée montrée sur les figures 5C, 6C et 7C, jusqu'à venir sur le bord d'extrémité 22A de la rampe, en ramenant l'embase 19 et en comprimant ainsi son ressort associé 21. Le bouton 6 occupe alors sa position initiale sous l'action de son ressort, contre le bord avant 7A.

Après l'armement de la canule, le praticien procède à celui de l'aiguille 4. Pour cela, comme le montrent les figures 5D, 6D et 7D, il recule à nouveau, à l'aide de son pouce, le bouton de commande 6 jusqu'à ce qu'il vienne en butée contre le bord arrière 7B de l'ouverture, entraînant l'étirement du ressort 23. Durant ce déplacement longitudinal arrière du bouton, comme le coulisseau 12 à canule est en position arrière, l'ergot 18 quitte le bord d'extrémité 22A de la rampe, puis suit son bord incliné par le déplacement transversal de son ressort 21 faisant coulisser son embase 19. Par ce décalage transversal perpendiculaire au plan P du dispositif, produit par le passage du ressort de son état comprimé à son état détendu, l'ergot 18 s'engage alors dans la butée coudée 16 du coulisseau 11 à aiguille et l'entraîne ainsi jusqu'à sa position arrière. Arrivé dans celle-ci, le crochet 11C de sa patte longitudinale 11B élastiquement déformable, engage la butée correspondante 2J du boîtier, de sorte que le coulisseau 11 est verrouillé dans sa position arrière, le ressort 14 étant comprimé et son aiguille 4 étant armée, revenue dans la canule.

Quand le praticien relâche le bouton 6, celui-ci est amené élastiquement via le ressort 23 vers sa position initiale, contre le bord avant 7A de l'ouverture 7, et l'ergot 18 s'applique à nouveau contre le bord d'extrémité 22A de la rampe 22, le ressort se comprimant en ramenant l'embase 19. Le dispositif 1 est alors en position armée, prêt au tir de prélèvement, comme le montrent les figures 5E, 6E et 7E.

Ainsi, l'armement du dispositif 1 s'effectue d'une seule main et de façon séquentielle, par deux déplacements successifs vers l'arrière du bouton de commande 6 qui agit, dans un premier temps, sur le coulisseau 12 à canule 5 puis, dans un second temps, sur le coulisseau 11 à aiguille 4.

Le praticien peut alors procéder au tir de prélèvement, comme le montrent les figures 5F, 5G et 5H.

Le tir peut être déclenché soit avec la gâchette avant 8, en faisant pivoter son levier 8A selon la flèche F1 de la figure 5F autour de l'axe 2K, soit avec la gâchette arrière 9, en poussant vers l'avant le bouton-poussoir 9A selon la flèche F2. Mais, au préalable, le praticien aura amené la gâchette avant 8 dans le plan longitudinal P-P, en agissant pour cela sur celle-ci selon la flèche F3 des figures 2 et 3, ce qui fait coulisser le levier 8A sur son axe 2K et l'éloigne de l'encoche 2L. La sécurité est alors effacée et le mécanisme de déclenchement est rendu opérationnel.

On voit sur la figure 5F que l'extrémité à pan incliné 9D de la patte longitudinale 9C du bouton-poussoir vient s'appliquer sur le crochet 11C de la patte élastique 11B du coulisseau 11 jusqu'à la déformer élastiquement et dégager le crochet 11C de la butée fixe correspondante 2J.

Comme le montre la figure 5G, sous l'action du ressort comprimé 14, le coulisseau 11 se déplace contre la patte ou butée intermédiaire fixe 2E, ce qui entraîne la sortie de l'aiguille et sa pénétration dans le tissu corporel (non représenté) du patient. On voit sur la figure 6G, que la butée 16 du coulisseau 11 est revenue dans sa position de départ, proche de l'ergot 18. Juste avant que le coulisseau 11 à aiguille n'atteigne sa butée 2E, la patte longitudinale avant 11D de ce coulisseau s'applique contre le crochet 12C de la patte élastiquement déformable 12B du coulisseau 12 à canule et agit sur celle-ci pour la dégager de sa butée 2H. Comme le montre la figure 5H, cela a pour effet d'entraîner le déplacement vers l'avant du coulisseau 12 à canule jusqu'à la butée avant fixe 2D, sous l'action du ressort comprimé 15 qui se détend, et la sortie de la canule 5 dans le tissu corporel, en entourant l'aiguille pour prélever l'échantillon tissulaire situé dans l'évidement 4A de l'aiguille 4. On voit sur la figure 6H que la butée 17 du coulisseau 12 est revenue au niveau du bord d'extrémité 22A de la rampe et de l'ergot 18.

Entre-temps, les gâchettes 8, 9 reviennent en position initiale sous l'action du ressort de rappel 9B. Le praticien retire alors les aiguille et canule du dispositif 1 du patient.

## Revendications

1. Dispositif pour prélever des échantillons corporels, du type comportant :
- une aiguille (4) dont l'extrémité distale forme un évidement apte à recevoir ledit échantillon ;
- une canule (5) entourant coaxialement ladite aiguille, lesdites aiguille et canule pouvant coulisser l'une par rapport à l'autre ;
- des coulisseaux (11, 12) associés respectivement auxdites aiguille et canule ;
- des ressorts (14, 15) associés respectivement auxdits coulisseaux ;
- un boîtier de préhension (2) de forme allongée, définissant un logement interne (10) à l'intérieur duquel sont agencés en série, selon un axe longitudinal dudit boîtier, lesdits coulisseaux (11, 12) pouvant coulisser entre une position avant dans le boîtier, pour laquelle lesdites aiguille (4) et canule (5) sont en position de repos, prêtes à être armées pour un tir de prélèvement, et une position arrière, pour laquelle lesdites aiguille et canule sont en position rétractée armée, prêtes pour ledit tir ;
- un bouton de commande (6) pour amener en position arrière lesdits coulisseaux à l'encontre desdits ressorts respectifs ;
- des moyens de blocage desdits coulisseaux (11, 12) en position arrière ; et
- un mécanisme de déclenchement (8, 9) pour effacer lesdits moyens de blocage et entraîner, sous l'action desdits ressorts, le déplacement en position avant desdits coulisseaux et le tir de prélèvement desdites aiguille et canule,
**caractérisé en ce que** lesdits coulisseaux (11, 12) comportent des butées (16, 17) qui sont transversalement décalées l'une par rapport à l'autre et **en ce que** ledit bouton de commande (6) comprend un ergot (18) mobile transversalement sous l'action de moyens de déplacement, et agissant séquentiellement sur lesdites butées décalées pour amener l'un après l'autre lesdits coulisseaux en position arrière.

2. Dispositif selon la revendication 1,
**caractérisé en ce que** lesdits moyens de déplacement comprennent un ressort (21) disposé transversalement entre ledit bouton (6) et ledit ergot (18) et permettant le passage de ce dernier d'une position rentrée pour laquelle l'un (11) desdits coulisseaux est déplacé en position arrière via sa butée (16), à une position sortie pour laquelle l'autre coulisseau (12) est déplacé en position arrière via sa butée décalée (17), et une rampe inclinée (22), prévue à l'intérieur dudit boîtier et qui ramène ledit ergot de sa position sortie vers sa position rentrée, lors du retour en position initiale dudit bouton.

3. Dispositif selon la revendication 2,
**caractérisé en ce que** ladite rampe inclinée (22) se termine par un bord d'extrémité latéral (22A) sur lequel s'applique, en position initiale dudit bouton, ledit ergot (18) comprimant son ressort, et qui est situé au même niveau que la butée (16) du coulisseau (11) à déplacer en premier.

4. Dispositif selon l'une quelconque des revendications 1 à 3,
**caractérisé en ce que** ledit ergot (18) est lié audit bouton (6) par une liaison à glissière (20) et peut coulisser transversalement, via celle-ci, sous l'action des moyens de déplacement.

5. Dispositif selon l'une quelconque des revendications 1 à 4,
**caractérisé en ce que** ledit coulisseau (12) à canule et son ressort (15) sont situés du côté avant dudit boîtier (2) et sont amenés en premier en position arrière, armée via ledit ergot, tandis que ledit coulisseau (11) à aiguille et son ressort (14) sont coaxialement situés du côté arrière et déplacés en second en position arrière, armée, le déplacement desdits coulisseaux et ressorts étant limité par des pattes fixes solidaires dudit boîtier.

6. Dispositif selon l'une quelconque des revendications 1 à 5,
**caractérisé en ce que** ledit bouton de commande (6) est monté longitudinalement coulissant à travers une ouverture oblongue (7) dudit boîtier et **en ce qu'**un ressort (23) disposé longitudinalement relie ledit boîtier audit bouton pour rappeler spontanément ce dernier dans sa position initiale, contre le bord avant correspondant de ladite ouverture.

7. Dispositif selon l'une quelconque des revendications 1 à 6,
**caractérisé en ce que** lesdits moyens de blocage comportent au moins une patte à crochet élastiquement déformable (11B, 11C - 12B, 12C) issue de chaque coulisseau, et une butée fixe correspondante (2H-2J) prévue à l'intérieur dudit boîtier et sur laquelle s'engage la patte à crochet du coulisseau correspondant quand celui-ci arrive en position arrière.

8. Dispositif selon l'une quelconque des revendications 1 à 7,
**caractérisé en ce que** ledit mécanisme de déclenchement dudit tir de prélèvement comporte, sur ledit boîtier, une gâchette avant (8) et une gâchette arrière (9) actionnables indépendamment l'une de l'autre et agissant sur lesdits moyens de blocage.

9. Dispositif selon la revendication 8,
**caractérisé en ce que** lesdites gâchettes avant et arrière sont reliées mécaniquement entre elles par une tige de liaison (24) située à l'intérieur dudit boîtier.

10. Dispositif selon l'une des revendications 8 et 9,
**caractérisé en ce que** ladite gâchette arrière (9) comprend un bouton-poussoir (9A) avec ressort de rappel (9B) et muni d'une patte (9C) agencée en saillie dans ledit boîtier pour libérer lesdits moyens de blocage (11B, 11C) dudit coulisseau à aiguille, et **en ce que** ledit coulisseau à aiguille (11) est équipé d'une patte de déblocage (11D) agencée en saillie pour agir sur lesdits moyens de blocage (12B, 12C, 2H) dudit coulisseau (12) à canule, par suite de son déplacement vers la position avant.

11. Dispositif selon l'une quelconque des revendications 8 à 10,
**caractérisé en ce que** ladite gâchette avant (8) comprend un levier pivotant (8A) autour d'un axe (2K) dudit boîtier orthogonal à son axe longitudinal, ladite tige de liaison (24) reliant ledit levier de la gâchette avant audit bouton-poussoir de la gâchette arrière.

12. Dispositif selon l'une quelconque des revendications 8 à 11,
**caractérisé en ce qu'**il comprend une sécurité pour rendre inopérationnel ledit mécanisme de déclenchement (8, 9), ladite sécurité consistant en une encoche (2L) ménagée dans ledit boîtier et dans laquelle peut être reçue ladite gâchette avant (8) par suite d'un déplacement transversal.

13. Dispositif selon l'une quelconque des revendications 1 à 12,
**caractérisé en ce que** ledit boîtier se compose de deux demi-coques assemblées entre elles le long d'un plan longitudinal.

## Claims

1. Device for taking samples from a body, of the type comprising:
- a needle (4) of which the distal end forms a recess able to receive said sample;
- a cannula (5) coaxially surrounding said needle, said needle and cannula being able to slide relative to one another;
- slides (11, 12) connected to said needle and cannula respectively;
- springs (14, 15) connected respectively to said slides;
- a grippable housing (2) of elongate shape, defining an inner seat (10) inside which are arranged in series, on a longitudinal axis of said housing, said slides (11, 12), which are able to slide between a front position in the housing, for which said needle (4) and cannula (5) are in a rest position and ready to be primed for taking a sample, and a rear position, for which said needle and cannula are in a primed, retracted position ready for said sampling;
- a control button (6) for bringing said slides into the rear position counter to said respective springs;
- means for blocking said slides (11, 12) in the rear position; and
- a trigger mechanism (8, 9) for cancelling said blocking means and, under the action of said springs, causing the forward displacement of said slides and the firing of said needle and cannula,
**characterised in that** said slides (11, 12) comprise limit stops (16, 17) which are transversely offset with respect to one another, and **in that** said control button (6) comprises a lug (18) which can be moved transversely under the action of displacement means and acts sequentially on said offset limit stops to bring said slides into the rear position one after the other.

2. Device according to claim 1, **characterised in that** said displacement means comprise a spring (21), arranged transversely between said button (6) and said lug (18) and permitting the latter to pass from a retracted position, for which one (11) of said slides is displaced into the rear position via the limit stop (16) thereof, to a deployed position, for which the other slide (12) is displaced into the rear position via the offset limit stop (17) thereof, and an inclined ramp (22), which is provided inside said housing and which returns said lug from the deployed position to the retracted position thereof upon the return of said button into the initial position.

3. Device according to claim 2, **characterised in that** said inclined ramp (22) terminates in a lateral end edge (22A), on which, in the initial position of said button, said lug (18) bears compressing the spring thereof, and which is situated at the same level as the limit stop (16) of the slide (11) which is to be displaced first.

4. Device according to any one of claims 1 to 3, **characterised in that** said lug (18) is connected to said button (6) by a slideway connection (20) and can slide transversely via said connection under the action of the displacement means.

5. Device according to any one of claims 1 to 4, **characterised in that** said slide (12) with cannula and the spring (15) thereof are located at the front of said housing (2) and are brought first into a rear, primed position via said lug, whereas said slide (11) with needle and the spring (14) thereof are located coaxially at the rear and are displaced second into the rear, primed position, the displacement of said slides and springs being limited by brackets fixed to said housing.

6. Device according to any one of claims 1 to 5, **characterised in that** said control button (6) is mounted so as to slide longitudinally through an oblong opening (7) of said housing, and **in that** a spring (23) which is arranged longitudinally connects said housing to said button in order to return the latter spontaneously to the initial position thereof, against the corresponding front edge of said opening.

7. Device according to any one of claims 1 to 6, **characterised in that** said blocking means comprise at least one bracket with a resiliently deformable hook (11B, 11C - 12B, 12C) issuing from each slide, and a corresponding fixed limit stop (2H-2J) which is provided inside said housing and on which the hooked bracket of the corresponding slide engages when said slide arrives in the rear position.

8. Device according to any one of claims 1 to 7, **characterised in that** said mechanism for triggering said sampling comprises, on said housing, a front tumbler (8) and a rear tumbler (9), which can be actuated independently of one another and act on said blocking means.

9. Device according to claim 8, **characterised in that** said front and rear tumblers are mechanically interconnected by a connection rod (24) situated inside said housing.

10. Device according to either claim 8 or claim 9, **characterised in that** said rear tumbler (9) comprises a push-button (9A) with a return spring (9B) and equipped with a bracket (9C) arranged so as to project into said housing in order to free said blocking means (11B, 11C) of said slide with needle, and **characterised in that** said slide (11) with needle is equipped with an unblocking bracket (11D) arranged so as to project in order to act on said blocking means (12B, 12C, 2H) of said slide (12) with cannula, following the displacement thereof into the front position.

11. Device according to any one of claims 8 to 10, **characterised in that** said front tumbler (8) comprises a lever (8A) pivoting about an axis (2K) of said housing orthogonal to the longitudinal axis thereof, said connection rod (24) connecting said lever of the front tumbler to said push-button of the rear tumbler.

12. Device according to any one of claims 8 to 11, **characterised in that** it comprises a safety means for rendering said trigger mechanism (8, 9) inoperative, said safety means consisting of a notch (2L) which is formed in said housing and in which said front tumbler (8) can be received following a transverse displacement.

13. Device according to any one of claims 1 to 12, **characterised in that** said housing is made up of two half-shells joined together along the longitudinal plane.

## Patentansprüche

1. Vorrichtung zur Entnahme von Proben aus dem Körper, die aufweist:
- eine Nadel (4), deren distales Ende eine Ausnehmung bildet, die zur Aufnahme der Probe geeignet ist;
- eine Kanüle (5), welche die Nadel koaxial umgibt, wobei die Nadel und die Kanüle zueinander verschiebbar sind;
- Gleiter (11, 12), die mit der Nadel bzw. mit der Kanüle verbunden sind;
- Federn (14, 15), die mit den betreffenden Gleitern verbunden sind;
- ein Gehäuse (2) von länglicher Form zum Ergreifen, das eine Innenaufnahme (10) vorgibt, in der in einer Längsachse des Gehäuses die Gleiter (11, 12) hintereinander angeordnet sind, die zwischen einer vorderen Position im Gehäuse, bei der sich die Nadel (4) und die Kanüle (5) in Ruhelagen befinden und bereit sind, für einen Entnahmeschuss gespannt zu werden, und einer hinteren Position gleiten können, bei der sich die Nadel und die Kanüle in einer zurückgezogenen, gespannten und zum Schuss bereiten Position befinden;
- einen Betätigungsknopf (6), der dazu dient, die Gleiter gegen die betreffenden Federn in die hintere Position zu bringen;
- eine Arretiereinrichtung zur Arretierung der Gleiter (11, 12) in der hinteren Position
und
- einen Auslösemechanismus (8, 9) zum Lösen der Arretiereinrichtung und zur Herbeiführung der Verschiebung der Gleiter unter der Wirkung der Federn in ihre vordere Position und des Entnahmeschusses der Nadel und der Kanüle,
**dadurch gekennzeichnet, dass**
die Gleiter (11, 12) Anschläge (16, 17) aufweisen, die in Querrichtung zueinander versetzt sind, und dass der Befestigungsknopf (6) einen Zapfen (18) aufweist, der unter der Einwirkung einer Verschiebungseinrichtung in Querrichtung beweglich ist und sequentiell auf die versetzten Anschläge so einwirkt, dass die Gleiter nacheinander in die hintere Position gebracht werden.

2. Vorrichtung nach Anspruch 1,
**dadurch gekennzeichnet, dass** die Verschiebungseinrichtung aufweist:
eine Feder (21), die zwischen dem Betätigungsknopf (6) und dem Zapfen (18) in Querrichtung angeordnet ist und die Umstellung des Zapfens (18) von einer zurückgezogenen Position, in der einer der Gleiter (11) über seinen Anschlag (16) in seine hintere Position verschoben ist, in eine Austrittsposition erlaubt, in welcher der andere Gleiter (12) über einen versetzten Anschlag (17) in die hintere Position verschoben ist, sowie eine schräge Auflauffläche (22), die im Inneren des Gehäuses vorgesehen ist und bei der Rückkehr des Betätigungsknopfes in seine Anfangsposition den Zapfen von seiner Austrittsposition in seine zurückgezogene Position zurückbringt.

3. Vorrichtung nach Anspruch 2,
**dadurch gekennzeichnet, dass** die schräge Auflauffläche (22) an ihrem Ende in einem seitlichen Rand (22A) endet, an dem in der Anfangsposition des Betätigungsknopfes der Zapfen (18) unter Zusammendrücken seiner Feder zur Anlage kommt und der sich auf gleicher Höhe wie der Anschlag (16) des zuerst zu verschiebenden Gleiters (11) befindet.

4. Vorrichtung nach einem der Ansprüche 1 bis 3,
**dadurch gekennzeichnet, dass** der Zapfen (18) durch eine Gleitverbindung (20) mit dem Betätigungsknopf (6) verbunden ist und durch diese unter der Einwirkung der Verschiebungseinrichtung in Querrichtung gleiten kann.

5. Vorrichtung nach einem der Ansprüche 1 bis 4,
**dadurch gekennzeichnet, dass**
sich der Gleiter (12) mit der Kanüle und seine Feder (15) auf der Vorderseite des Gehäuses (2) befinden und zunächst in die hintere Position gebracht werden, die über den Zapfen gespannt ist, während sich der Gleiter (11) mit der Nadel und seine Feder (14) koaxial auf der hinteren Seite befinden und danach in die hintere, gespannte Position gebracht werden, wobei die Verschiebung der Gleiter und der Federn durch mit dem Gehäuse verbundene Laschen begrenzt ist.

6. Vorrichtung nach einem der Ansprüche 1 bis 5,
**dadurch gekennzeichnet, dass** der Betätigungsknopf (6) so angebracht ist, dass er durch eine längliche Öffnung (7) des Gehäuses gleiten kann, und dass eine in Längsrichtung vorgesehene Feder (23) das Gehäuse mit dem Betätigungsknopf verbindet, um diesen spontan in seine Ausgangsposition in Anlage am entsprechenden vorderen Rand der Öffnung zurückzubringen.

7. Vorrichtung nach einem der Ansprüche 1 bis 6,
**dadurch gekennzeichnet, dass** die Arretiereinrichtung mindestens eine elastisch biegbare Hakenlasche (11B, 11C - 12B, 12C), die aus jedem Gleiter heraussteht, sowie einen entsprechenden festen Anschlag (2H - 2J) aufweist, der im Inneren des Gehäuses vorgesehen ist und an dem die Hakenlaschen des entsprechenden Gleiters zum Eingriff kommen, wenn der Gleiter in seine hintere Position gelangt.

8. Vorrichtung nach einem der Ansprüche 1 bis 7,
**dadurch gekennzeichnet, dass** der Auslösemechanismus für den Entnahmeschuss einen vorderen Drücker (8) und einen hinteren Drücker (9) am Gehäuse aufweist, die unabhängig voneinander betätigbar sind und auf die Arretiereinrichtung wirken.

9. Vorrichtung nach Anspruch 8,
**dadurch gekennzeichnet, dass** der vordere Drücker und der hintere Drücker durch eine Verbindungsstange (24), die sich im Inneren des Gehäuses befindet, mechanisch miteinander verbunden sind.

10. Vorrichtung nach Anspruch 8 oder 9,
**dadurch gekennzeichnet, dass** der hintere Drücker (9) eine Drucktaste (9A) mit einer Rückholfeder (9B) aufweist, wobei die Drucktaste mit einer Lasche (9C) versehen ist, die im Gehäuse vorstehend vorgesehen ist, um die Arretiereinrichtung (11B, 11 C) des Gleiters mit der Nadel freizugeben, sowie dadurch, dass der Gleiter (11) mit der Nadel mit einer Entarretierungslasche (11D) versehen ist, die vorspringend angeordnet ist und dazu dient, auf die Arretiereinrichtung (12B, 12C, 2H) des Gleiters (12) mit der Kanüle nach seiner Verschiebung zur vorderen Position zu wirken.

11. Vorrichtung nach einem der Ansprüche 8 bis 10,
**dadurch gekennzeichnet, dass** der vordere Drücker (8) einen Hebel (8A) aufweist, der um eine Achse (2K) des Gehäuses, die senkrecht zu seiner Längsachse angeordnet ist, schwenkbar ist, wobei die Verbindungsstange (24) den Hebel des vorderen Drückers mit der Drucktaste des hinteren Drückers verbindet.

12. Vorrichtung nach einem der Ansprüche 8 bis 11,
**dadurch gekennzeichnet, dass** sie eine Sicherung aufweist, mit welcher der Auslösemechanismus (8, 9) außer Betrieb gesetzt werden kann, wobei die Sicherung aus einer Ausnehmung (2L) besteht, die im Gehäuse vorgesehen ist und in welcher der vordere Drücker (8) nach einer Verschiebung in Querrichtung aufgenommen werden kann.

13. Vorrichtung nach einem der Ansprüche 1 bis 12,
**dadurch gekennzeichnet, dass** das Gehäuse aus zwei Halbschalen zusammengesetzt ist, die längs einer Längsebene zusammengefügt sind.
